(19) Europäisches Patentamt
European Patent Office
Office européen des brevets

(11) Publication number: 0 145 739 B1

(12) **EUROPEAN PATENT SPECIFICATION**

(45) Date of publication of patent specification: **08.02.89**

(51) Int. Cl.[4]: **C 07 F 9/65,** A 61 K 31/675

(21) Application number: **84902178.7**

(22) Date of filing: **14.05.84**

(86) International application number: **PCT/US84/00737**

(87) International publication number: **WO 84/04748 06.12.84 Gazette 84/28**

# (54) NOVEL ANTIVIRAL AGENTS.

(30) Priority: **24.05.83 US 497720**

(43) Date of publication of application: **26.06.85 Bulletin 85/26**

(45) Publication of the grant of the patent: **08.02.89 Bulletin 89/06**

(84) Designated Contracting States: **FR**

(56) References cited:
EP-A-0 049 072
EP-A-0 074 306
DE-A-2 009 834
DE-A-3 045 375
FR-A-2 381 781
GB-A-1 243 213
US-A-3 446 793
US-A-3 560 478

Journal of Medicinal Chemistry, vol. 22, no. 1, January 1979, J.A. Montgomery et al.: "Phosphonate analogue of 2'-Deoxy-5-fluorouridylic acid", pp. 109-111

(73) Proprietor: **SRI INTERNATIONAL**
**333 Ravenswood Avenue**
**Menlo Park, California 94025 (US)**

(72) Inventor: **REIST, Elmer, Joseph**
**581 Berkeley Avenue**
**Menlo Park, CA 94025 (US)**
Inventor: **STURM, Priscilla, Anna**
**1549 Montalvo Drive**
**Mountain View, CA 94040 (US)**

(74) Representative: **Rinuy, Santarelli**
**14, avenue de la Grande Armée**
**F-75017 Paris (FR)**

(56) References cited:
Journal of Heterocyclic Chemistry, vol. XI, February, April, June 1974, J.A. Montgomery et al.: "The use of the wittig reaction in the modification of purine nucleosides (1)", pp. 211-218

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 145 739 B1

(56) References cited:
Journal of the American Chemical Society, vol. 92, no. 18, 9 September 1970, G.H. Jones: "Communications to the editor. Synthesis of Isosteric Phosphonate analogs of some biologically important phosphodiesters", pp. 5510-5511

Journal of the American Chemical Society, vol. 90, no. 19, 11 September 1968, G.H. Jones et al.: "The Synthesis of 6'-Deoxyhomonucleoside-6'-phosphonic Acids", pp. 5337-5338

Liebigs Annalen der Chemie, vol. 1, 1984, Verlag Chemie (Weinheim, DE), J. Hollmann et al.: "Darstellung und Konformationszuordnung einiger 5'-homologer Adenosinderivate", pp. 98-107

Chemical Review, no. 3, June 1977, R. Engel: "Phosphonates as analogues of natural phosphates", pp. 349-367

Biochemistry, vol. 12, no. 9, 24 April 1973, A. Hampton et al.: "Synthesis of homoadenosine-6'-phosphonic acid and studies of its substrate and inhibitor properties with adenosine monophosphate utilizing enzymes", pp. 1730-1736

## Description

Field of the Invention

This invention concerns nucleotide analogues and their synthesis and use. More particularly, it concerns phosphonic acid analogues of natural and synthetic nucleoside phosphates and their preparation and use as antiviral agents.

Background of the Invention

There is a recognized need for antiviral agents. Herpes virus hominis alone infects between 50 and 150 million Americans at this time. A number of the antiviral agents that are currently viewed as most effective against herpes are nucleoside analogues. These materials include iododeoxyuridine, 2-hydroxyethoxymethylguanine, 2′-fluoro-5-iodo-1-arabinofuranosyl cytosine and 5-E-bromovinyldeoxyuridine. It is believed that these materials act through their conversion by viral thymidine kinase (but not by host TK) to the nucleotide which is then converted to the triphosphate and incorporated into viral DNA. The incorporation of these analogues into the viral DNA prevents its replication and thus is lethal to the virus. Two shortcomings of this antiviral mechanism have been recognized, however. First, thymidine kinase negative herpes mutants ($TK^-$) have been identified which are inherently inactive toward phosphorylating these analogues and thus permitting their incorporation in viral DNA. In addition, $TK^+$ mutants that are resistant to 2-hydroxyethoxymethylguanine have been reported in mice by H. Field, et al, in *J Infect Dis*, *143* 281 (1981). $TK^+$ mutants resistant to iododeoxyuridine have been reported in humans by A. Hirano, et al, in *Acta Virol 23* 226 (1979). It may be that these newly-discovered resistant viral strains do not undergo the monophosphorylation or triphosphate formation needed to permit incorporation in the DNA.

References to these antiviral agents of the art and their use include *Am J Med*, *73* No 1A, July 20, 1982 "Proceedings of a Symposium on Acyclovir"; *Biochem Biophys Acta*, *32* 295—6 (1959); *Antimicrob Agents Chemother*, 578—584 (1965); *Sience*, *145* 585—6 (1964); *Science*, *255* 468—80 (1975); *J. Med Chem*, *19 495—8 (1976); Proc Natl Acad Sci*, *76* 4415—18 (1979); and *J Med Chem 22* 21—24 (1979).

The present invention provides antiviral materials which can be lethally incorporated into DNA without the dependence upon enzyme-moderated phosphorylation. The materials of the invention are phosphonate analogues of the mono-, di- and triphosphates of the deoxynucleotide analogs. An article by Robert Engel appearing at *Chem Reviews*, *11*, #3 pp 349—367 (1977) discusses phosphonate analoges of nucleotides and the like. Other representative references in this area, some of which are cited in the *Chem Reviews* article, are German O.L.S. 2,350,608 (1974) of Syntex (Jones and Moffatt inventors); German O.L.S. 2,009,834 1970 also of Syntex with Jones and Moffatt as inventors; British Patent 1,243,214 of Syntex, and US Patent 3,560,478 of Myers.

Statement of the Invention

A group of new materials have now been found. These materials in a broad sense are phosphonate analogues of mono-, di-, and triphosphates of antiviral nucleoside analogues. These analogues differ from nonlethal natural nucleosides by variations in their sugar ribose moiety and/or by variations in their nucleoside base moieties. Such materials are represented stucturally as

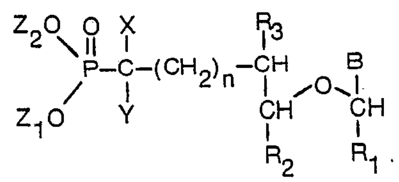

wherein $Z_1$ and $Z_2$ are the same or different and selected from the group made up of hydrogen, the one to six carbon alkyls, phenyl and benzyl, X is H, OH, or together with Y = O, Y is H or together with X = O, n is an integer, 0, 2 or 4, $R_1$ is H and $R_2$ is H or $—CH_2OH$, $R_3$ is H or OH and B is a substituted or unsubstituted purine base.

In other aspects this invention relates to the preparation of these materials, their formulation into antiviral pharmaceutical compositions and the use of these formulations to treat viral infections, in particular herpes infections.

### Detailed Description of the Invention

The compounds

The compounds of this invention are phosphonates which have the structure set forth above in Statement of the Invention.

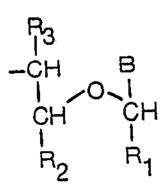

The unit defines an antiviral nucleoside.

In this structure, $Z_1$ and $Z_2$ preferably are each selected from hydrogens and one to four carbon alkyls. More preferably $Z_1$ and $Z_2$ are each hydrogens. Further in this structure, the integer n is significant in defining whether the compound is in size equivalent to a nucleoside monophosphate, a diphosphate or a triphosphate.

Preferred bases include guanine and adenine.

Preferred nucleoside analogues (e.g.

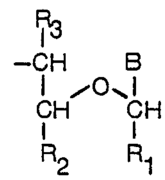

include phosphonates (and di- and triphosphate-phosphonate analogues) having the following general structures

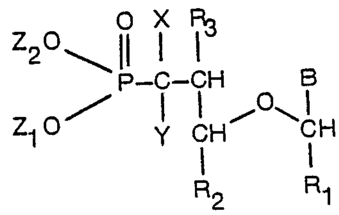

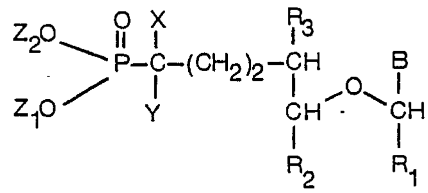

and

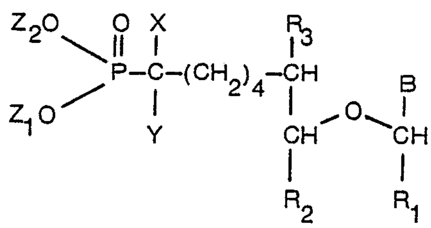

wherein X, Y, $Z_1$, $Z_2$, B, $R_1$, $R_2$ and $R_3$ are as described above. In preferred embodiments, X is hydrogen or hydroxyl and Y is hydrogen or X together with Y is oxygen. Thus, representative compounds can have the structures shown in Table I.

TABLE I

Representative Structures

| Structure | Structure Number |
|---|---|
| 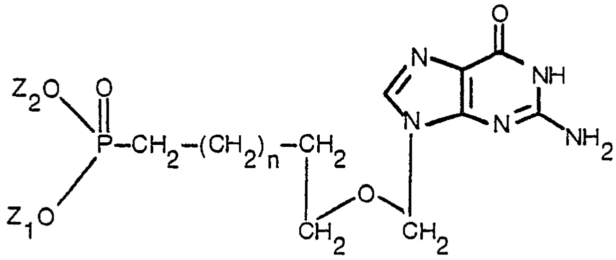 | 1 |
| 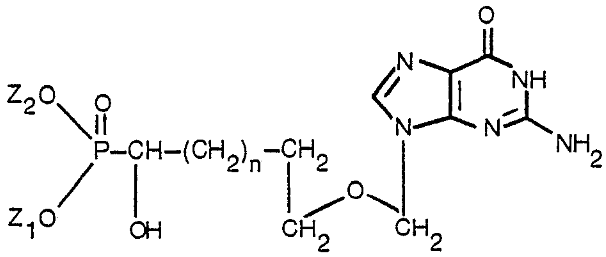 | 2 |
| 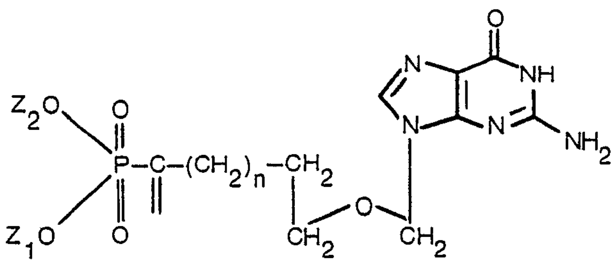 | 3 |
| 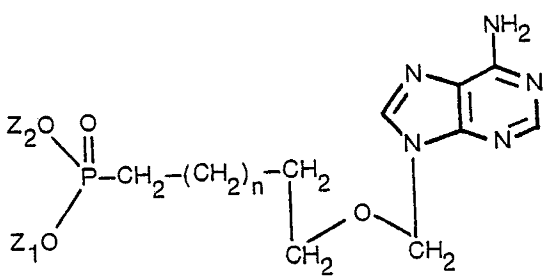 | 12 |
| 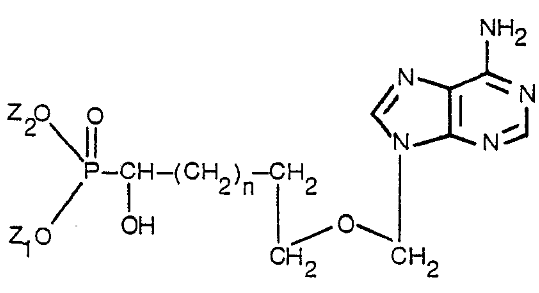 | 13 |

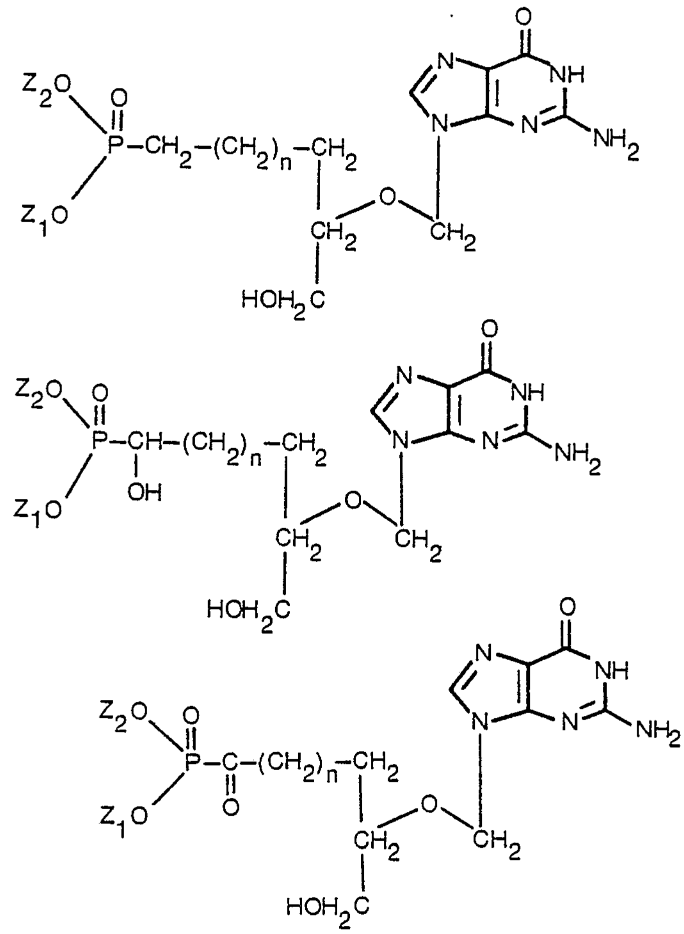

20

21

22

Table II

Representative Compounds

Monophosphate Analogues

| Compound Number | Structure Number | $Z_1$ | $Z_2$ | n |
|---|---|---|---|---|
| 1a | 1 | H | H | 0 |
| 1b | 1 | $CH_3$ | $CH_3$ | 0 |
| 1c | 1 | H | $CH_3$ | 0 |
| 1d | 1 | H | $C_2H_5$ | 0 |
| 2a | 2 | H | H | 0 |
| 2b | 2 | $CH_3$ | $CH_3$ | 0 |
| 2c | 2 | H | $CH_3$ | 0 |
| 2d | 2 | H | $C_2H_5$ | 0 |
| 3a | 3 | H | H | 0 |
| 3b | 3 | $CH_3$ | $CH_3$ | 0 |
| 3c | 3 | H | $CH_3$ | 0 |
| 3d | 3 | H | $C_2H_5$ | 0 |
| 22a | 22 | H | H | 0 |
| 22b | 22 | $CH_3$ | $CH_3$ | 0 |
| 22c | 22 | H | $CH_3$ | 0 |
| 22d | 22 | H | $C_2H_5$ | 0 |

Diphosphate Analogs

| Compound Number | Structure Number | $Z_1$ | $Z_2$ | n |
|---|---|---|---|---|
| 1e | 1 | H | H | 2 |
| 1f | 1 | $CH_3$ | $CH_3$ | 2 |
| 1g | 1 | H | $CH_3$ | 2 |
| 1h | 1 | H | $C_2H_5$ | 2 |
| 2e | 2 | H | H | 2 |
| 2f | 2 | $CH_3$ | $CH_3$ | 2 |
| 2g | 2 | H | $CH_3$ | 2 |
| 2h | 2 | H | $C_2H_5$ | 2 |
| 22a | 22 | H | H | 2 |
| 22b | 22 | $CH_3$ | $CH_3$ | 2 |
| 22c | 22 | H | $CH_3$ | 2 |
| 22d | 22 | H | $C_2H_5$ | 2 |

Triphosphate Analogues

| Compound Number | Structure Number | $Z_1$ | $Z_2$ | n |
|---|---|---|---|---|
| 1 | 1 | H | H | 4 |
| 1 | 1 | $CH_3$ | $CH_3$ | 4 |
| 1 | 1 | H | $CH_3$ | 4 |
| 1 | 1 | H | $CH_3$ | 4 |
| 22 | 22 | H | H | 4 |
| 22 | 22 | $CH_3$ | $CH_3$ | 4 |
| 22 | 22 | H | $CH_3$ | 4 |
| 22 | 22 | H | $C_2H_5$ | 4 |

These are merely representative compounds as it will be apparent to those skilled in the art that other combinations of substituents and bases could be employed as well.

Preparation

The compounds of this invention can be prepared by the following general procedures: The materials having structures *1, 2, 12, 13, 20, 21,* and *22* in Table I can be made with the representative reaction sequence I.

I.

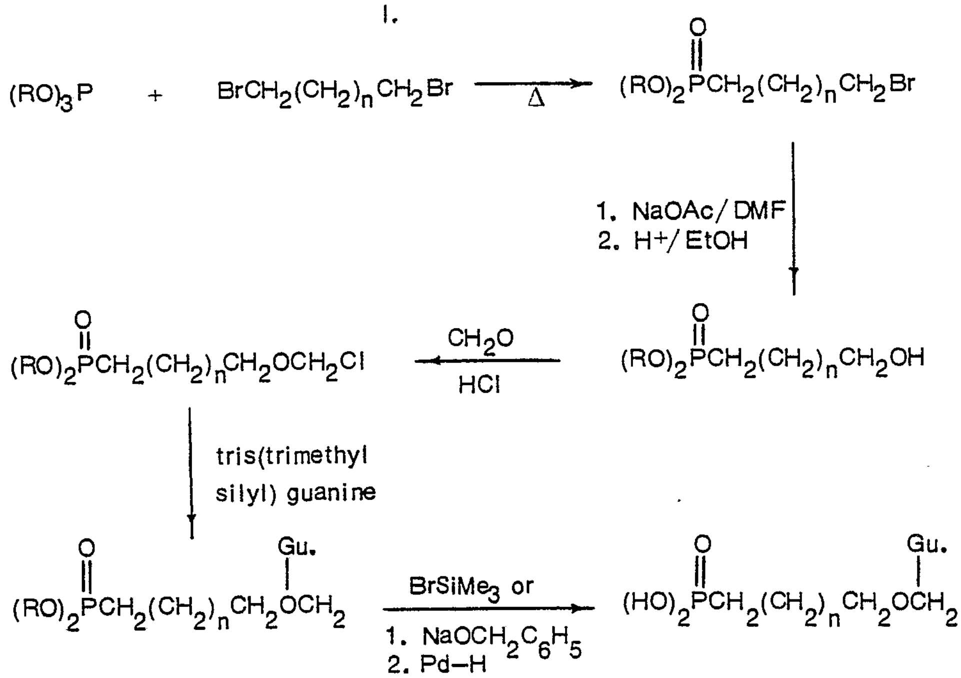


SCHEME I

In this sequence, a trialkyl ($C_2$, $C_4$ or $C_6$) phosphite reacts with a dibromoalkane in an Arbuzov reaction to give the bromoalkyl phosphonate (See *J Am Chem Soc, 87* (2), 253 (1965)). (All cited references are incorporated herein).

Displacement of the bromide using sodium acetate in DMF followed by hydrolysis of the acetate ester gives diethyl 3-hydroxypropylphosphonate (i.e. in sequence I, $R = C_2H_5$, $n = 1$). This material is chloromethylated to the chloromethylester and then reacted with a suitably protected base such as *tris*-trimethylsilylguanine by the method of Kelley, et al, *J Med Chem, 24* 1528 (1981). This yields the phosphono product, e.g. 9-(3-phosphonopropyloxymethyl)guanine, directly. Phosphonate esters are smoothly cleaved by bromotrimethylsilane to the phosphonic ester by the method of McKenna, et al, *Tet Letts*, 155 (1977).

Oxygen functionality α to the phosphonate can be introduced by the chemistry outlined in Scheme IV. The appropriate propargyl phosphonic acid (13) can be prepared from tetrahydropyranyl-propargyl alcohol (12) by the procedures outlined by Chattha and Aquiar, *J Org Chem, 36* 2719—20 (1971). Selective hydrogenation to the vinyl phosphonate followed by the sequence outlined in Scheme II results in phosphonate analogues with one or two hydroxyls in the phosphonate chain.

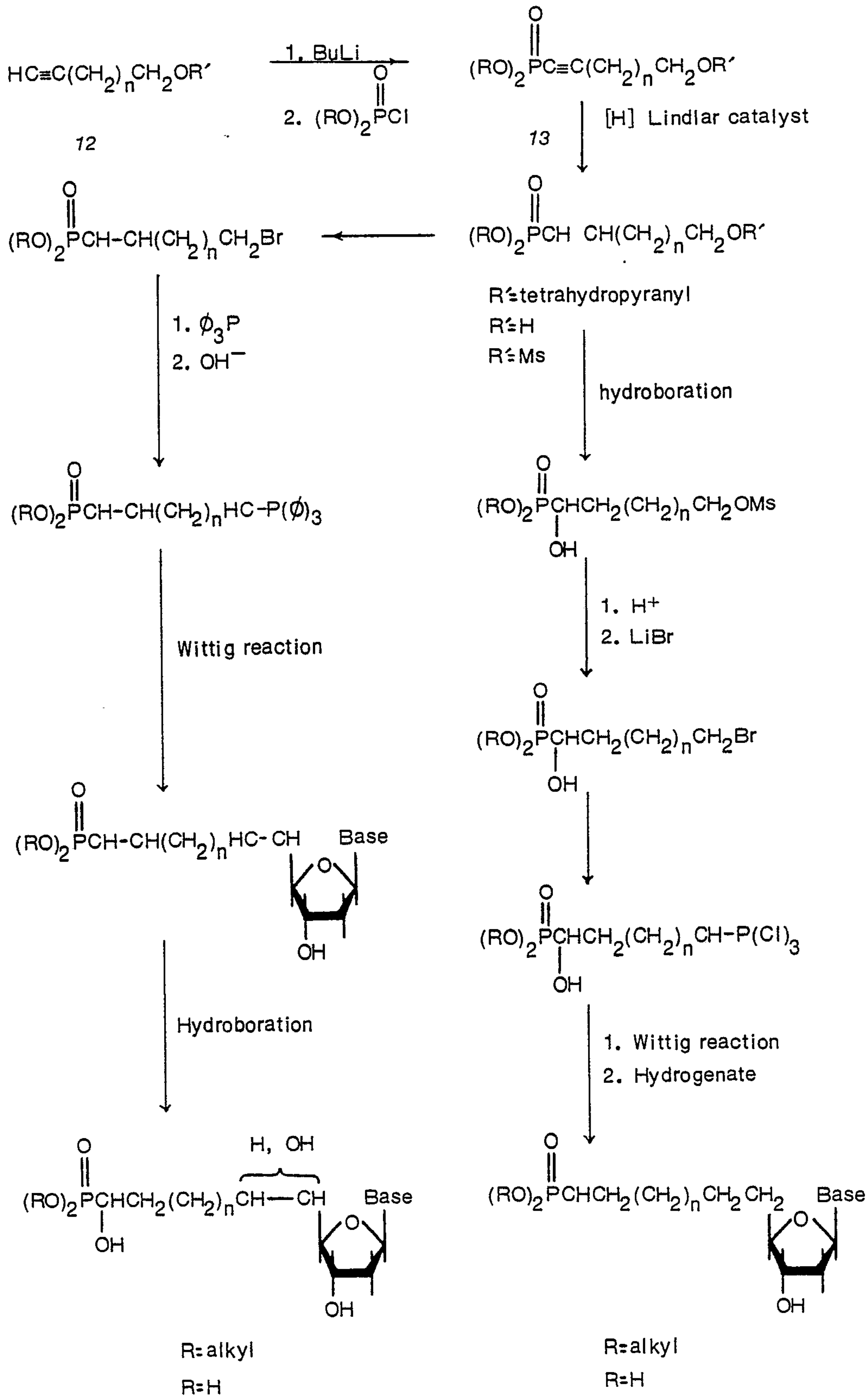


SCHEME II*

* (to be applied to the claimed subject–matter "mutatis mutandis")

Carbonyl analogs can be prepared by the chemistry outlined in Scheme III. This method involves Arbuzov reaction of triethylphosphite with β-acetoxypropionyl chloride (*14*) by the method of Yamashita, et al, *Bull Chem Soc Japan*, *53*(6) 1625 (1980).

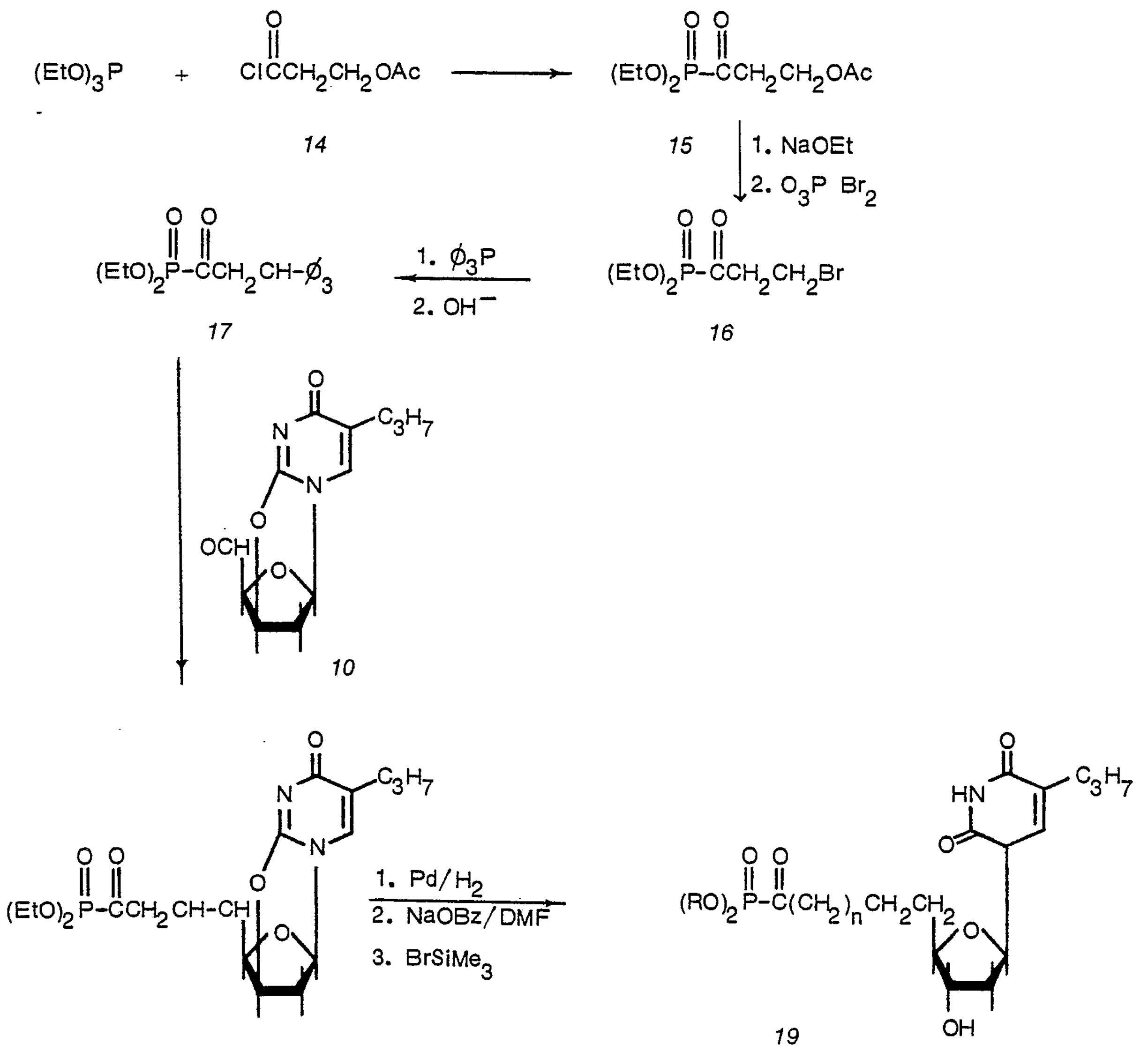


*14* *15* *16* *17* *10* *18* *19*

R=Et, n=1

R=H, n=1

SCHEME III*

* (to be applied to the claimed subject-matter "mutatis mutandis")

This gives the α-carbonyl phosphonate ester (*15*). Conversion of *15* to the bromoethyl derivative *16* followed by reaction with triphenylphosphine will give the Wittig reagent (*17*). Condensation of *17* with the appropriate aldehyde (e.g. 10) gives the olefin *18*, which, after hydrogenation and deblocking, results in the desired product *19*, a nucleoside diphosphonate analog. The nucleoside triphosphate analog *19* (wherein n = 3) can be prepared starting from 5-acetoxyvalerylchloride.

Salts

Physiologically acceptable salts of compounds of this invention are prepared by methods known in the art. The salts include ammonium salts and salts of physiologically acceptable metals, particularly $Li^+$, $K^+$, $Na^+$, $Ca^{++}$ and $Mg^{++}$, and are novel compounds and comprise a further aspect of the invention. Metal salts can be prepared by reacting a metal hydroxide with a compound of the invention. Examples of metal salts which can be prepared in this way are salts containing $Li^+$, $Na^+$, and $K^+$. A less soluble metal salt can be

precipitated from a solution of a more soluble salt by addition of a suitable metal compound. Acid salts can be prepared by reacting a compound of the invention with an acid such as HCl, HBr, $H_2SO_4$, or an organic sulphonic acid.

Pharmaceutical Preparations

The compounds of this invention (including the physiologically acceptable salts thereof) have antiviral activity. They present activity against Herpes Simplex viruses and related viruses for example Herpes Simplex virus I, Herpes Simplex virus II, Epstein-Barr virus, varicella Zoster virus, and cytomegalo virus. Thus the compounds can be formulated into pharmaceutical preparations. Such preparations are composed of one or more of the compounds in association with a pharmaceutically acceptable carrier. The book *Remington's Pharmaceutical Sciences, 15th Ed* by E. W. Martin (Mark Publ. Co., 1975) discloses typical carriers and methods of preparation, which disclosure is incorporated by reference.

The compounds may be administered topically, orally, parenterally (e.g. intravenously, by intramuscular injection, or by intraperitoneal injection or the like depending upon the nature of the viral infection being treated.

For internal infections the compositions are administered orally or parenterally at dose levels of about 0.1 to 300 mg/kg, preferably 1.0 to 30 mg/kg of mammal body weight and can be used in man in a unit dosage form administered one to four times daily in the amount of 1 to 250 mg per unit dose. For oral administration, fine powders or granules may contain diluting, dispersing and/or surface active agents, and may be presented in water or in a syrup, in capsules or sachets in the dry state or in a nonaqueous solution or suspension, wherein suspending agents may be included; in tablets, wherein binders and lubricants may be included, or in a suspension in water or a syrup. Where desirable or necessary, flavoring, preserving, suspending, thickening or emulsifying agents may be included. Tablets and granules are preferred oral administration forms and these may be coated.

For parenteral administration or for administration as drops, as for eye infections, the compounds may be presented in aqueous solution in a concentration of from about 0.1 to 10%, more preferably about 0.1 to 7%. The solution may contain antioxidants, buffers, etc.

Alternatively, for infections of the eye, or other external tissues, e.g. mouth and skin the compositions are preferably applied to the infected part of the body of the patient topically as an ointment, cream, aerosol or powder, preferably as an ointment or cream. The compounds may be presented in an ointment, for instance with a water soluble ointment base, or in a cream, for instance with an oil in water cream base in a concentration of from about 0.01 to 10%, preferably 0.1 to 7%, most preferably about 0.5% w/v. Additionally, viral infections of the eye, such as Herpetic keratitus may be treated by use of a sustained release drug delivery system as is described in the art.

The exact regimen for administration of the compounds and compositions disclosed herein will necessarily be dependent upon the needs of the individual subject being treated, the type of treatment and, of course, the judgement of the attending practitioner.

The invention will be further described by the following nonlimiting examples.

EXAMPLE I

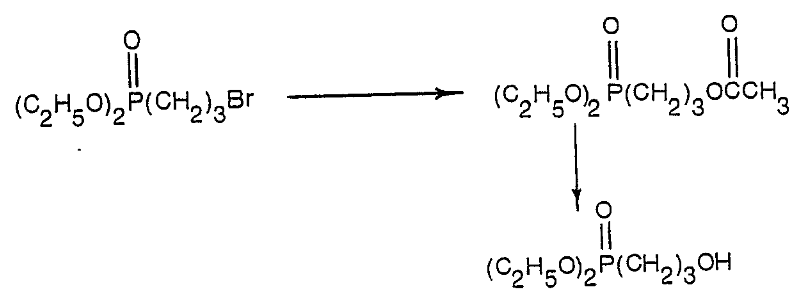

Diethyl-3-hydroxypropylphosphonate

Diethyl-3-bromophosphonate (12.0 g, 46 mmol, prepared by the method of Anatol Eberhard and F. H. Westheimer, *JACS 87* 253-260 (1965)) was stirred with 12.0 g NaOAc.$3H_2O$ in 125 ml DMF heated in a steam bath. The reaction was evaporated to dryness in vacuo after 2 hours and partitioned between $H_2O$ and EtOAc, extracting the aqueous layer five times. The ethyl acetate extract was washed once with brine, dried with $Na_2SO_4$, filtered, and evaporated to dryness in vacuo to yield 9.8 g light yellow oil (89%). $^1H$ NMR ($CDCl_3$) δ 1.3 (tr, 6H), 1.5—2.0 (m, 4H), 2.03 (s, 3H), 4.1 (assym. quintet, 6H); thin layer chromatography on SiGF developed with 2:1 EtOAc:$CH_2Cl_2$ gave $R_f$ 0.30. The isolated diethyl-3-acetoxypropylphosphonate (9.8 g, 41 mmol) in 200 ml abs. EtOH was stirred with 30 ml Dowex 50 ($H^+$) which had been rinsed three times each with $H_2O$ and EtOH. After 4½ days at room temperature, another 10 ml of similarly prepared resin was added. Six hours later, the reaction was filtered and evaporated in vacuo. The quantitative yield of yellow oil was purified by dry column chromatography on 400 g silica packed in a 2.75 inch flat diameter nylon

tube. The column was eluted with 1:9 MeOH:EtOAc and the appropriate fractions were cut and slurried with 1:1 MeOH:EtOAc. Filtration and evaporation *in vacuo* afforded 5.33 g (66%) pale yellow oil. $^1H$ NMR ($CDCl_3$ $D_2O$): δ 1.30 (tr, 6H), 1.60—2.08 (m, 4H), 3.67 (tr, 2H), 4.13 (quintet, 4H); thin layer chromatography on SiGF developed with 1:9 MeOH:EtOAc gave an $R_f$ of 0.57.

9(3-phosphono-1-propyloxymethyl)guanine

To 9.40 mmol silated guanine (James L. Kelley, Mark P. Krochmal, and Howard J. Shaeffer, *J Med Chem, 24* 1528—1531 (1981)) in 9 ml dry toluene was added 7.60 mmol diethyl-3-chloromethoxypropylphosphonate, prepared from diethyl-3-hydroxypropylphosphonate according to the procedure of Kelley, et al, followed by the addition of 2.2 ml triethylamine. The reaction was refluxed 24 hours and evaporated to dryness in vacuo. The residue was digested with 70 ml EtOH and the voluminous tan solid was isolated by suction filtration. The solid was dissolved in water, made basic with conc. $NH_4OH$, and treated with excess aqueous lead diacetate. The lead salt was isolated by centrifugation and dissolved in 50% acetic acid followed by treatment with $H_2S$ for 20 minutes. The black lead sulfide was removed by suction filtration through Celite. The filtrate was evaporated to dryness in vacuo, triturated in EtOH, and filtered. The residue was further triturated in DMF and filtered to yield 320 mg off-white solid. This solid was dissolved in minimum water, acidified with 1 M HCl. Thereafter it was neutralized with 1 M NaOH and lyophilized. The solid residue was triturated in a mixture of DMF, $H_2O$, and EtOH and filtered to yield 276 mgh of 9(3-phosphono-1-propyloxymethyl) guanine as a white solid (8.3%). Anal. ($C_9H_{12}N_5O_5P \cdot 2Na \cdot 5H_2O$) C, H, N; UV $\gamma_{max}$ (ε): pH 1, 255 (14, 700); pH 7, 251 (15, 600); pH 11, 257 (12, 800), 267 (12, 800); mass spectrum (TMS derivative) m/e 591 ($M^+$ of $TMS_4$ derivative); $^1H$ NMR ($D_2O$): δ 1.25—1.90 (M, 4H), 3.42 (tr, 2H), 5.50 (s, 2H), 7.92 δ (s, 1H). Thin layer chromatography on SiGF developed with 7:3 $CH_3CN$:0.1 N $NH_4Cl$ gave $R_f$ 0.20.

EXAMPLE II

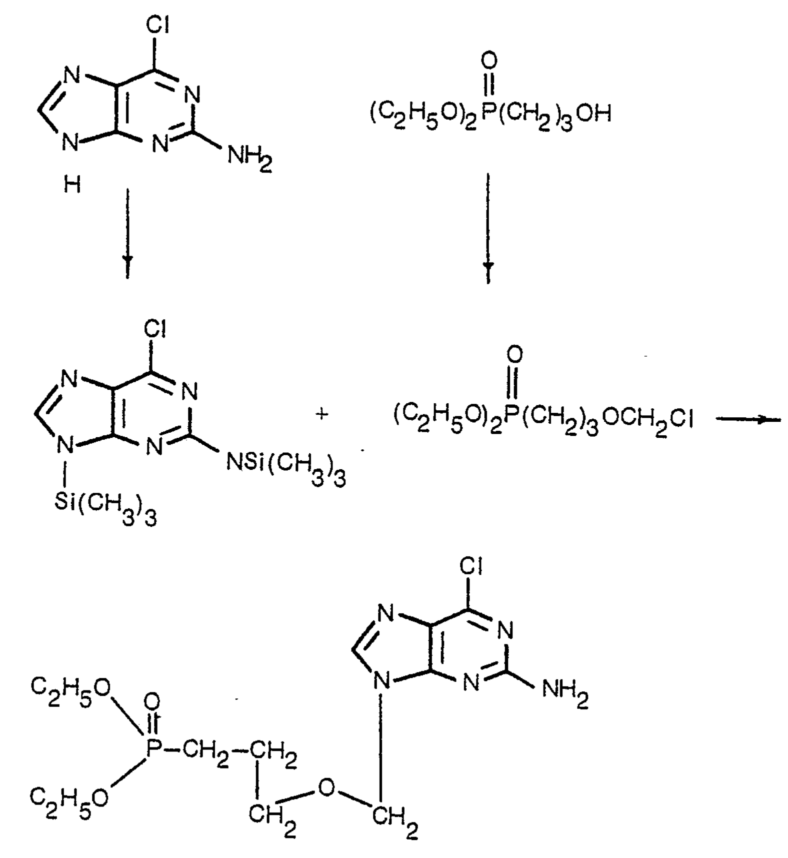

6-Chloro-9(3-diethylphosphono-1-propyloxymethyl)guanine

To 0.5 g (2.95 mmol) 2-amino-6-chloropurine, silated and treated with $Hg(CN)_2$ according to the procedure of Robins and Hatfield (Morris J. Robins and Peter W. Hatfield, *Can J Chem, 60* 547—553 (1982)) in 40 ml benzene was added a solution of 2.68 mmol diethyl-3-chloromethoxypropylphosphonate prepared from (0.525 g) diethyl-3-hydroxypropylphosphonate according to the procedure of Kelley, et al, (James L. Kelley, Mark P. Krochlam and Howard J. Shaeffer, *J Med Chem, 24* 1528—1531 (1981)). The reaction was refluxed for 2 hours, cooled and 400 ml $CHCl_3$ was added. The organic phase was washed successively with 80 ml each of aqueous saturated $NaHCO_3$ and 1M aqueous KI. The organic solution was dried over $Na_2SO_4$, filtered and evaporated to 790 mg of yellow gum. A portion of this crude material was used to conduct

hydrolysis experiments. The remaining material was chromatographed on a silica column. A solution of 574 mg of the crude reaction product was placed on 20 g silica packed in a column using 5:3 EtOAc: PrOH. Elution with the same mixed solvent afforded sixteen fractions of 10—20 ml each. Fractions 7—12 were combined to yield 258 mg of a colorless oil which spontaneously crystallized. Trituration in $CH_2Cl_2 \cdot Et_2O$ afforded two crops of white solid (207 mg), mp 109—110° (28%). A yield of 46% was obtained from a reaction performed on 45.2 mmol of the starting purine. Anal ($C_{13}H_{21}ClN_5O_4P$) $C_1H_1N$; UV $\gamma_{max}$ (ε): pH 1, 246 (ε 6600), 310 (7200); pH 7, 247 (6800), 308 (7400); pH 11 247 (6600), 308 (7100); mass spectrum: m/e 377 ($M^+$); $^1H$ NMR ($CDCl_3$): δ 1.3 (tr, 6H), 1.52—2.18 (m, 4H), 3.58 (tr, 2H), 4.09 (qu, 4H), 5.48 (s. with broad base, 4H), 7.89 (s, 1H). Thin layer chromatography on SiGF developed with 5:3 EtOAc:nPrOH gave $R_f$ 0.40.

9(3-ethylphosphono-1-propyloxymethyl)guanine

6-Chloro-9(3-diethylphosphono-1-propyloxymethyl)guanine (75 mg; 0.2 mmol) was combined with 5 ml 1N aqueous NaOH and refluxed 1 hour. The cooled reaction was neutralized with Dowex® 50X8 (pyridinium form) and filtered, rinsing liberally with water. The solution was partially evaporated to remove pyridine and was then lyophilized. The orange-colored residue (74 mg) was redissolved in $H_2O$ and centrifuged to remove insoluble material. The decanted solution (2 ml) was chromatographed on a 0.9 × 46 cm column of Whatman DE—52 Cellulose, $HCO_3$ form, using a linear gradient of one liter each $H_2O$ and 0.2 M $NH_4HCO_3$ after an initial $H_2O$ elution. Fractions (7 ml each) 43—47 yielded 25 mg (36%) of fluffy white solid after three lyophilizations. Electron impact mass spectrum (TMS derivative) showed m/l 547 ($M^+$ of TMS derivative); chemical ionization mass spectrum (TMS derivative) showed m/l 548 ($M^+$ + H of $TMS_3$ derivative). $^1H$ NMR ($D_2O$) showed δ 1.19 (tr, 3H), 1.4—1.9 (m, 4H), 3.59 (tr, 2H), 3.90 (quintet, 2H), 5.47 (s, 2H), 8.2 (br·s, 1H). Thin layer chromatography behavior on SiGF: Rf 0.40 when developed with 7:3 $CH_3CH$. 0.1 N aqueous $NH_4Cl$. The material had a formula of ($C_{11}H_{18}N_5O_5P \cdot H_2O$) Calc: C—37.82% H—5.77%, N—20.0. Found: C—38.27%, H—5.84%, N—19.65%. A UV spectrum was run on the material and showed UV $\gamma_{max}$ (ε): pH 1, 256 (10, 400), 278 shoulder; pH 7, 252 (11, 300) 271 shoulder; pH 11, 256—258 (9, 600) 267 shoulder. The product was relyophilized.

EXAMPLE III

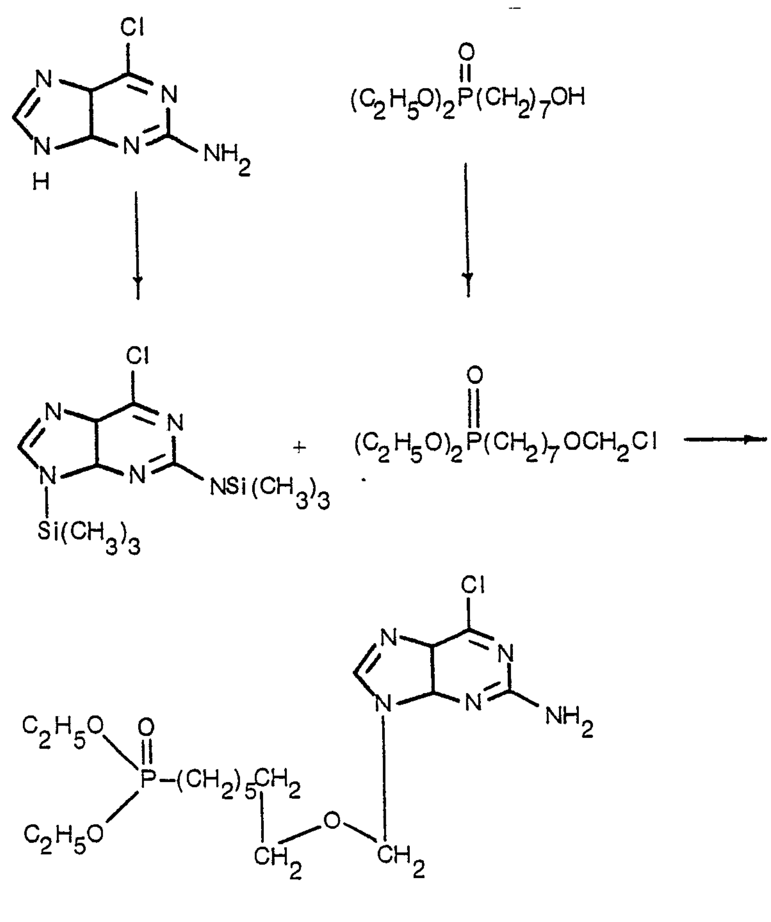

6-Chloro-9(7-diethylphosphono-1-heptyl-methyl)guanine

Diethyl 7-chloromethoxyheptylphosphonate was prepared from 1,7-dibromoheptane and triethylphosphite by the procedures used to prepare diethyl 3-chloromethoxypropylphosphonate (Example

I). It was reacted with silated 2-amino-6-chloropurine, and mercuric cyanide as described for the preparation of 6-chloro-9(3-diethylphosphono-1-propyloxymethyl)guanine (Example II) to give 32% of product as a colorless gum. UV $\gamma_{max}$ pH 1: 246 nm (ε 6220), 310 nm (ε 6380); $\gamma_{max}$ pH 7, 247 nm (ε 5910), 310 nm (ε 6410); $\gamma_{max}$ pH 11, 246 nm (ε 5950), 309 nm (ε 6380); mass spectrum $^1$H NMR ($CDCl_3$) ∫ 1.1—1.9 (m, 18H), 3.48 (t, 2H), 4.10 (q, 4H), 5.47 (s, 2H), 5.88 (s, 2H), 7.93 (s, 1H). Thin layer chromatography on silica gel GF gave $R_f$ 0.15 using ethylacetate:ethanol (100:1).

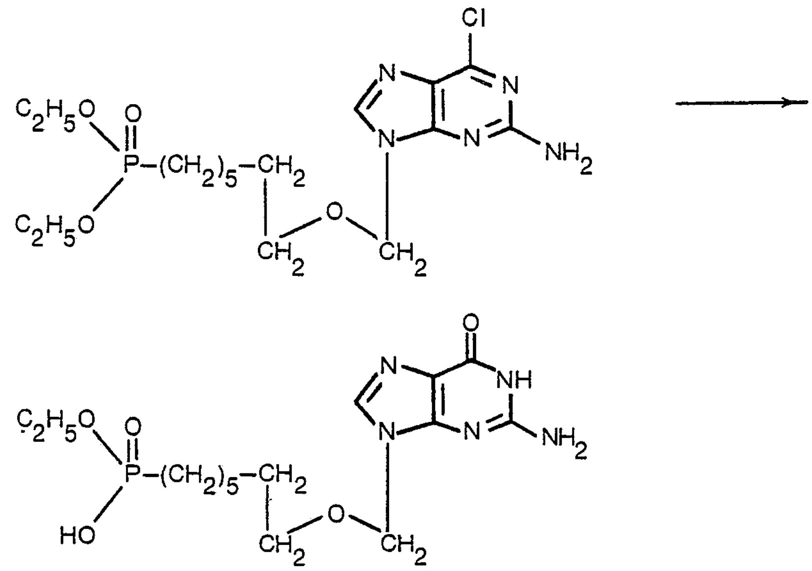

9-(7-ethylphosphono-1-heptyloxymethyl)guanine:

6-chloro-9(7-diethylphosphono-1-heptyloxy-methyl)guanine was hydrolyzed by refluxing 1 N aqueous sodium hydroxide for 4 hours and isolated in 30% yield as described for the preparation of 9-(3-ethylphosphono-1-propyloxymethyl)guanine (Example II). It had $R_f$ 0.5 on silica gel GF using acetonitrile (7:3) 0.1 N aqueous ammonium chloride. Proton NMR ($D_2O$) 1.1—1.5 (m, 15H), 3.5 (t, 2H), 3.90 (q, 2H), 5.45 (s, 2H) UV.

Biological Testing

The compounds of Example I and II were evaluated *in vitro* as antiviral agents against herpes virus.

The virus strain employed was Strain McCrae of type 1 herpes (thymidine kinase positive virus) prepared and titered in MA—104 cells and frozen at −90°C until use.

Continuous passaged monkey kidney (MA—104) cells were used, with growth medium consisting of Minimum Essential Medium (MEM) supplemented with 0.1% $NaHCO_3$ and 9% fetal calf serum. Test medium consisted of MEM supplemented with 2% fetal bovine serum, 0.18% $NaHCO_3$ and 50 µl gentamicin.

The last compounds were added to test medium at a concentration of 2000 µg/ml for use as a positive control.

Antiviral Test Method

To a 96 well microtiter plate containing an established 24 hour monolayer of cells from which the medium has been decanted was added 0.1 ml of varying (one-half $\log_{10}$) concentrations of test compound, which incubated on the cell 15 minutes, after which 0.1 ml of virus in a concentration of 320 cell culture 50% infectious doses (CCID50)/0.1 ml was added. The plate was covered with plastic wrap and incubated at 37°C. Included with the test were toxicity controls (each concentration of compound + test medium in place of virus), virus controls (virus + test medium in place of compound) and cell controls (test medium in place of compound and virus). The cells were examined microscopically after 72 hours for evidence of cytotoxicity and for viral cytopathic effect (CPE). Vidarabine was run on the same plate in parallel.

Antiviral activity was determined by observation of inhibition of viral CPE. This activity was expressed by minimum inhibitory concentration (MIC), defined as that dose range of compound causing 50% CPE inhibition. A Virus Rating (VR) was also determined, which is a numerical expression of antiviral activity, weighted to take into account any cytotoxicity observed. Generally, a VR of 0.1—0.4 is usually indicative of slight antiviral effect, 0.5—0.9 indicates moderate antiviral effect, and ≥1.0 implies strong antiviral activity.

The results of these are summarized in tables A and B. The test compound had a strong activity against the thymidine kinase positive type I herpes virus. The activity was considered equivalent to that of vidarabine.

TABLE A

Effect of compound of Example I and Vidarabine on Thymidine Kinase-Positive Type 1 Herpes Virus Infections in MA-104 Cells

| Compound of I | | Vidarabine | |
|---|---|---|---|
| Conc. (μg/ml | CPE[a] Inhib. (%) | Conc. (μ/ml) | CPE[a] Inhib. (%) |
| 1000 | 100 | 1000 | 100 |
| 320 | 94 | 320 | 100 |
| 100 | 79 | 100 | 87 |
| 32 | 62 | 32 | 87 |
| 10 | 49 | 10 | 69 |
| 3.2 | 28 | 3.2 | 28 |
| 1.0 | 31 | 1.0 | 56 |
| 0 | | 0 | |
| VR[b] | 1.4 | 1.3 | |
| MIC[c] | 10 | 10 | |

[a]Cytopathic effect, % cell alteration or destruction.

[b]Virus rating, a numerical expression of antiviral activity (Sidwell et al, Appl Microbiol, 22:797, (1971), 0.1 - 0.4 = slight activity, 0.5 - 0.9 = moderate activity, ≥ 1.0 = strong activity.

[c]Minimum inhibitory concentration - that dosage range wherein a 50% CPE inhibition is seen.

TABLE B

Effect of compound of Example II and Vidarabine on Thymidine Kinase Positive Type 1 Herpes Virus in MA-104 Cells (Two Tests)

| Compound of II | | | Vidarabine (Control) | | |
|---|---|---|---|---|---|
| Conc. (μg/ml | Test1 CPE[a] Inhib. (%) | Test2 CPE[a] Inhib. % | Conc. (μ/ml) | Test1 CPE[a] Inhib. (%) | Test2 CPE[a] Inhib. (%) |
| 1000 | 100 | 76 | 1000 | 100 | 100 |
| 320 | 82 | 67 | 320 | 100 | 100 |
| 100 | 47 | 57 | 100 | 96 | 85 |
| 32 | 6 | 57 | 32 | 96 | 57 |
| 10 | 38 | 48 | 10 | 60 | 39 |
| 3.2 | 96 | 52 | 3.2 | 2 | 0 |
| 1.2 | 69 | 48 | 1.0 | 0 | 0 |
| 0 | | | 0 | | |
| VR[b] | >2.0 | >1.4 | VR[b] | 0.8 | 07 |
| MIC[c] | <1.0 | <1.0 | MIC[c] | 10 | 10 |
| MTD[d] | 320 | >1000 | | 10 | 10 |

[a]Cytopathic effect, % cell alteration or destruction.

[b]Virus rating, a numerical expression of antiviral activity (Sidwell et al, Appl Microbiol 22:797, (1971), 0.1 - 0.4 = slight activity, 0.5 - 0.9 = moderate activity, ≥ 1.0 = strong activity.

[c]Minimum inhibitory concentration - that dosage range wherein a 50% CPE inhibition is seen, μg/ml.

[d]Maximum tolerated dose, μg/ml.

The compound of Example II was further evaluated by the above-described *in vitro* test method against cytomegalo virus. The compound was strongly active with a VR of 2.1—2.3.

The compound of Example II was tested *in vivo* in guinea pigs as an agent against thymidine kinase positive herpes virus. The animals were innoculated with the virus. Eighteen hours later the material of Example II (0.4% solution in water or 1—2% solution), a 5% solution of acyclovir or a 1.4% solution of poly(vinylalcohol) was administered and five days later blister diameters at the point of innoculation were measured. Satellite lesions were measured as well.

The results of these tests are given in Table C and shown that the compound has superior activity against $TK^+$ virus.

TABLE C

Effect of Compound of Example II
*in vivo* against Herpes Virus

| Virus | Placebo, 1.4% poly(vinylalcohol) | Acyclovir, 5% | Cx of II 1-2%* | Cx of II 0.4% |
|---|---|---|---|---|
| $TK^+$ | 1.7** | 1.0 | 0.9 | 2.1 |
| Satellite lesions | 9 | 4 | 6 | 11 |

*saturated solution

**average number of lesions

Formulations

The following formulations based on the compounds of the invention and their preparation are representative.

A formulation suitable for injections intramuscularly or intraperitoneally is prepared by combining the first four of the following materials

| | |
|---|---|
| Compound of the Invention | 1 gram |
| Poly(ethylene glycol) | 50 grams |
| Propylene glycol | 50 grams |
| Tween 80® suspension agent | 1.5 grams |
| Injectable Saline | 200 ml |

and then adding the last material. The material forms a clear solution which is filtered and sealed in sterile containers.

A simple intravenous injection formulation is formed by dissolving 1 gram of an active compound in 250 ml of injectable saline which after filtering is packaged in sterile bottles.

A cream for topical administration is formulated by stirring 10 g of active compound of the invention with 20 g of mineral oil, 40 g of petroleum jelly, 0.3 g of mixed methyl/propyl paraben and 5 g of nonionic surfactant at 50°C. Then 150 ml of water are stirred into the mixture at 50°C at high speed to form a cream and the mixture is cooled and packaged in capped tubes.

An oral dosage form is prepared from 10 g of compound of the invention, 100 g of lactose, and 1 g of starch which are mixed with 0.1 g of magnesium stearate in methanol to granulate. The methanol is removed by gentle heating with stirring. A portion of this material is retained as a granular powder for oral use while the remainder is hand formed into 250 mg tablets in a manual tableting machine.

The foregoing examples and formulations have been presented to illustrate the present invention and are not to be construed as limitations on the invention's scope which is instead defined by the following claims.

**Claims**

1. A compound having the structure

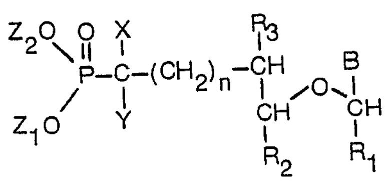

wherein $Z_1$ and $Z_2$ are the same or different and selected from the group made up of hydrogen, the one to six carbon alkyls, phenyl and benzyl, X is H, OH, or together with Y = O, Y is H or together with X = O, n is an integer, 0, 2 or 4, $R_1$ is H and $R_2$ is H or $—CH_2OH$, $R_3$ is H or OH and B is a substituted or unsubstituted purine base.

2. The compound of claim 1 wherein $R_1$ is hydrogen and $R_2$ is hydrogen.

3. The compound of claim 1 wherein $R_1$ is hydrogen and $R_2$ is hydroxymethyl and $R_3$ is hydrogen.

4. The compound of any one of claims 1, 2 or 3 wherein $Z_1$ and $Z_2$ are each selected from the group made up of hydrogen and one to four carbon alkyls.

5. The compound of claim 4 wherein X and Y are each hydrogens.

6. The compound of claim 4 wherein X and Y are =O.

7. The compound of claim 4 wherein X is hydroxyl and Y is hydrogen.

8. The compound of any one of claims 1 to 5 wherein n is 0.

9. The compound of any one of claims 1 to 5 wherein n is 2.

10. The compound of any one of claims 1 to 5 wherein n is 4.

11. A metal salt of a compound of claim 1.

12. A pharmaceutical preparation comprising a compound of claim 1 in a pharmaceutically acceptable carrier.

13. A compound according to any one of claims 1 to 11 for use in treating herpes in mammals.

14. A compound according to claim 1 which is 9(3'-phosphono-1'-propyloxymethyl)-guanine.

15. A compound according to claim 1 which is 9(3'-ethylphosphono-1'-propyloxymethyl)-guanine.

16. A compound according to claim 1 wherein X is H, Y is H, $R_1$ is H, $R_2$ is H, $R_2$ is —$CH_2OH$, $R_3$ is H and B is 9'-guanine.

## Patentansprüche

1. Verbindung mit der Struktur

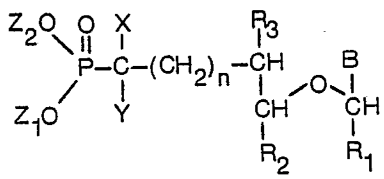

worin $Z_1$ und $Z_2$ identisch oder unterschiedlich und aus der Gruppe, die aus Wasserstoff, den Alkylen mit einem bis sechs Kohlenstoffatomen, Phenyl und Benzyl besteht, ausgewählt sind, X H, OH oder zusammen mit Y = O ist, Y H oder zusammen mit X = O ist, n eine ganze Zahl, 0, 2 oder 4 ist, $R_1$ H ist und $R_2$ H oder —$CH_2OH$ ist, $R_3$ H oder OH ist und B eine substituierte oder unsubstituierte Purinbase ist.

2. Verbindung nach Anspruch 1, worin $R_1$ Wasserstoff ist und $R_2$ Wasserstoff ist.

3. Verbindung nach Anspruch 1, worin $R_1$ Wasserstoff ist und $R_2$ Hydroxymethyl ist und $R_3$ Wasserstoff ist.

4. Verbindung nach einem beliebigen der Ansprüche 1, 2 oder 3, worin $Z_1$ und $Z_2$ jeweils aus der Gruppe ausgewählt sind, die aus Wasserstoff und Alkylen mit einem bis vier Kohlenstoffatomen besteht.

5. Verbindung nach Anspruch 4, worin X und Y jeweils Wasserstoff sind.

6. Verbindung nach Anspruch 4, worin X und Y =O bedeuten.

7. Verbindung nach Anspruch 4, worin X Hydroxyl ist und Y Wasserstoff ist.

8. Verbindung nach einem beliebigen der Ansprüche 1 bis 5, worin n gleich 0 ist.

9. Verbindung nach einem beliebigen der Ansprüche 1 bis 5, worin n gleich 2 ist.

10. Verbindung nach einem beliebigen der Ansprüche 1 bis 5, worin n gleich 4 ist.

11. Metallsalz einer Verbindung nach Anspruch 1.

12. Pharmazeutische Zubereitung, die eine Verbindung nach Anspruch 1 in einem pharmazeutisch annehmbaren Träger umfaßt.

13. Verbindung nach einem beliebigen der Ansprüche 1 bis 11 für die Verwendung beim Behandeln von Herpes in Säugern.

14. Verbindung nach Anspruch 1, die 9(3'-Phosphono-1'-propyloxymethyl)-guanin ist.

15. Verbindung nach Anspruch 1, die 9(3'-Ethylphosphono-1'-propyloxymethyl)-guanin ist.

16. Verbindung nach Anspruch 1, worin X H ist, Y H ist, $R_1$ H ist, $R_2$ H ist, $R_2$ —$CH_2OH$ ist, $R_3$ H ist und B 9'-Guanin ist.

## Revendications

1. Composé répondant à la formule structurale

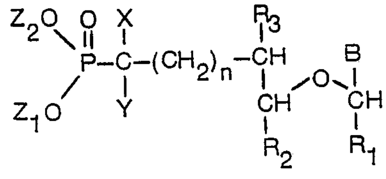

dans laquelle $Z_1$ et $Z_2$ sont identiques ou différents et sont choisis dans le groupe comprenant l'hydrogène, les groupes alkyle ayant un à six atomes de carbone, phényle et benzyle, X représente H, OH ou, conjointement avec Y, représente O, Y représente H ou, conjointement avec X, représente O, n est le nombre entier 0, 2 ou 4, $R_1$ représente H et $R_2$ représente H ou un groupe $-CH_2OH$, $R_3$ représente H ou un groupe OH et B est une base purique substituée ou non substituée.

2. Composé suivant la revendication 1, dans lequel $R_1$ représente l'hydrogène et $R_2$ représente l'hydrogène.

3. Composé suivant la revendication 1, dans lequel $R_1$ représente l'hydrogène, $R_2$ représente un groupe hydroxyméthyle et $R_3$ représente l'hydrogène.

4. Composé suivant l'une quelconque des revendications 1, 2 ou 3, dans lequel $Z_1$ et $Z_2$ sont choisis chacun dans le groupe comprenant l'hydrogène et des groupes alkyle de un à quatre atomes de carbone.

5. Composé suivant la revendication 4, dans lequel X et Y représentent chacun l'hydrogène.

6. Composé suivant la revendication 4, dans lequel X et Y forment conjointement un groupe O.

7. Composé suivant la revendication 4, dans lequel X représente un groupe hydroxyle et Y représente l'hydrogène.

8. Composé suivant l'une quelconque des revendications 1 à 5, dans lequel n a la valeur 0.

9. Composé suivant l'une quelconque des revendications 1 à 5, dans lequel n a la valeur 2.

10. Composé suivant l'une quelconque des revendications 1 à 5, dans lequel n a la valeur 4.

11. Sel métallique d'un composé suivant la revendication 1.

12. Préparation pharmaceutique comprenant un composé suivant la revendication 1, dans un support pharmaceutiquement acceptable.

13. Composé suivant l'une quelconque des revendications 1 à 11, destiné à être utilisé dans le traitement de l'herpès chez les mammifères.

14. Composé suivant la revendication 1, qui est la 9(3'-phosphono-1'-propyloxyméthyl)-guanine.

15. Composé suivant la revendication 1, qui est la 9(3'-éthylphosphono-1'-propyloxyméthyl)-guanine.

16. Composé suivant la revendication 1, dans lequel X représente H, Y représente H, $R_1$ représente H, $R_2$ représente H ou un groupe $-CH_2OH$, $R_3$ représente H et B est la 9'-guanine.